**Europäisches Patentamt**

**European Patent Office**

**Office européen des brevets**

(11) Veröffentlichungsnummer : **0 290 017 B1**

(12) **EUROPÄISCHE PATENTSCHRIFT**

(45) Veröffentlichungstag der Patentschrift :
**15.03.95 Patentblatt 95/11**

(51) Int. Cl.⁶ : **G01N 33/546, // G01N33/68**

(21) Anmeldenummer : **88107221.9**

(22) Anmeldetag : **05.05.88**

(54) **Verfahren zur quantitativen Bestimmung von Serumproteinen in Körperflüssigkeiten und Mittel zur Durchführung des Verfahrens.**

(30) Priorität : **08.05.87 DE 3715333**

(43) Veröffentlichungstag der Anmeldung :
**09.11.88 Patentblatt 88/45**

(45) Bekanntmachung des Hinweises auf die Patenterteilung :
**15.03.95 Patentblatt 95/11**

(84) Benannte Vertragsstaaten :
**AT BE CH DE ES FR GB IT LI LU NL SE**

(56) Entgegenhaltungen :
**EP-A- 0 080 614**
**EP-A- 0 083 869**
**EP-A- 0 087 728**

(56) Entgegenhaltungen :
**DE-A- 2 126 128**
**GB-A- 2 052 059**
**PATENT ABSTRACTS OF JAPAN, Band 9, Nr. 186 (P-377)(1909), 02 August 1985; & JP-A-60 057 255**

(73) Patentinhaber : **BEHRINGWERKE Aktiengesellschaft**
**Postfach 1140**
**D-35001 Marburg (DE)**

(72) Erfinder : **Kapmeyer, Wolfgang, Dr.**
**Reinhardswaldstrasse 5**
**D-3550 Marburg (DE)**
Erfinder : **Toth, Tibor, Dr.**
**Gründeberg 1**
**D-3550 Marburg (DE)**

## Beschreibung

Die Erfindung betrifft ein Verfahren zum Nachweis oder zur Bestimmung eines Partners einer partikelverstärkten immunologischen Reaktion nach einem nephelometrischen, turbidimetrischen oder Partikelzählverfahren, ein dafür geeignetes Mittel und die Verwendung von Antiserum mit einer von der immunologischen Reaktion abweichenden Spezifität in einem solchen Verfahren.

Es ist bekannt, die Empfindlichkeit serologischer oder immunologischer Bestimmungsverfahren durch die Verwendung von Indikator- oder Trägerteilchen zu erhöhen, die mit dem entsprechenden immunologischen Reagenz (einem Antikörper oder Antigen) beladen sind. Als Trägermaterial können zum Beispiel rote Blutkörperchen oder Zellen einer Zellkultur verwendet werden. Auch Latexpartikel mit einem Durchmesser von 0,02 bis 5 μm werden hierfür eingesetzt.

Ein solcher "partikelverstärkter" nephelometrischer oder turbidimetrischer Test kann Proteine bis zu Konzentrationen von etwa 5 ng/ml sicher nachweisen. Bei einem derartigen partikelverstärkten Test werden an partikuläre Polymeren gebundene Antikörper bzw. Antigene ("Festphase") eingesetzt. Zur Bestimmung eines Antigens kommt ein Festphasen-gebundener Antikörper zum Einsatz, zur Bestimmung eines Antikörpers ein Festphasen-gebundenes Antigen. In beiden Fällen erfolgt durch die Immunreaktion eine Agglutination der Polymerpartikel. Daraus resultiert eine Größenzunahme der Agglutinate und das Streulichtsignal bzw. die Trübung des Reaktionsansatzes nimmt zu.

Patent Abstract of Japan (Band 9, Nr. 186 (P-377)[1909] 2, August 1985) beschreibt die Unterdrückung unspezifischer Reaktionen von Serumproben beim Agglutinationsverfahren durch Zusatz von Hitze-degeneriertem IgG.

In Dokument DE-A-2 126 128 wird dazu gamma-Globulin nicht-menschlichen Ursprungs benutzt.

GB-A-2 052 059 verwendet Nicht-Immunseren derjenigen Tierspezies als Hemmsubstanz, welche für die Herstellung der Nachweisantikörper verwendet wurde.

EP-A-0 083 869 benutzt als Hemmsubstanz Antikörper-fragmente spezieller Art.

Aus der EP 0 087 728 ist ein Latex-Agglutinationsverfahren unter Verwendung von Gammaglobulinen ohne Antikörperspezifität bezüglich eines an der Umsetzung beteiligten Reaktionspartners bekannt. Diese werden durch Mischen eines Latexreagenzes mit der nachzuweisenden Probe in einem Tropfen auf einem Glas- oder Kunststoffplättchen durchgeführt. Eine positive Reaktion bewirkt eine Zusammenballung und Ausflockung des Latexreagenzes, und das milchige Aussehen der Latexsuspension verschwindet. Derartige Verfahren erlauben eine qualitative Aussage. Sie sind subjektiv durch das Auge, aber nicht durch ein optisches System auswertbar. Außerdem sind sie nicht automatisierbar.

Aus der Europäischen Patentanmeldung EP-A-0080 614 sind Latexpartikel bekannt, die über Säureamidgruppen gebundene Acetal-Funktionen enthalten. Derartige Partikel lassen sich zur nephelometrischen Bestimmung von C-reaktivem Protein einsetzen. Dazu werden Serumproben mit Puffer verdünnt, normalerweise 1:100, wodurch der Einfluß störender Serumproteine, die andernfalls zu falschen Ergebnissen führen würden, vernachlässigbar wird. Diese Arbeitsweise ist möglich, weil für diagnostische Zwecke im allgemeinen Konzentrationen an C-reaktivem Protein von mehr als 5 mg/l vorliegen müssen. Will man aber die Konzentration von Spurenproteinen im Bereich von 1 μg/l bis 5 μg/l messen, dann dürfen die Proben nicht entsprechend mit Puffer verdünnt werden, weil sonst die Konzentration des nachzuweisenden Proteins so gering wird, daß die Nachweisempfindlichkeit nicht ausreicht. In der EP-A 0 080 614 wird die Verwendung von TWEEN (0.2 % und 1.7 %) beschrieben.

Eine Steigerung der Nachweisempfindlichkeit bei Latexpräparationen nach dem Stand der Technik aus nur beispielsweise 1:5 verdünnten Seren ist jedoch nicht ohne weiteres möglich und liefert zum Beispiel für die Bestimmung von Alpha-Fetoprotein (AFP) oder Immunglobulin E keinen zufriedenstellend funktionierenden Test.

Beim partikelverstärkten nephelometrischen oder turbidimetrischen Test finden Immunreaktionen an Festphasen statt. Es ist eine Eigenschaft dieser Festphasen, unspezifisch Proteine aus den untersuchten Körperflüssigkeiten zu adsorbieren, die stören können. Eine andere Schwierigkeit, die in derartigen Methoden auftreten kann, besteht darin, daß das menschliche Serum das Protein C1q und Rheumafaktoren (RF) enthält. Diese binden sich an Antikörper. Außerdem können die Mengen an Rheumafaktor und C1q in menschlichen Seren in weiten Grenzen variieren, weshalb es normalerweise erforderlich ist, die Seren vorher einer Behandlung zur Inaktivierung von C1q oder Entfernung von Rheumafaktoren zu unterziehen. Andernfalls resultieren falsche Konzentrationswerte für Spurenproteine in Körperflüssigkeiten. Eine sichere Bestimmung mit Hilfe von nephelometrischen oder turbidimetrischen Verfahren ist daher nicht möglich.

Der in der EP 0087 728 für visuell ablesbare Latexagglutinationsverfahren vorgeschlagene Zusatz von Gammaglobulinen ohne Antikörperspezifität bezüglich eines an der Umsetzung beteiligten Reaktionspartners ist für nephelometrische und turbidimetrische Messungen nicht wirksam genug, um auch die Störung von Se-

2

ren mit hohem Gehalt an Rheumafaktoren zu unterdrücken.

Es wurde nun überraschenderweise gefunden, daß die vorstehend genannten Schwierigkeiten, verursacht durch eine nicht-spezifische Agglutination der Partikel in einem solchen Testsystem verhindert werden können, indem der Test in Gegenwart eines verdünnten Antiserums, das beziehungsweise die weder mit dem zu bestimmenden Antigen oder Antikörper noch mit dem an die Partikel gebundenen Partner reagieren, sowie in Gegenwart von ᴿTween 20 durchgeführt wird.

Gegenstand der Erfindung ist ein Verfahren zum Nachweis oder zur Bestimmung eines Partners einer partikelverstärkten immunologischen Reaktion nach einem nephelometrischen, turbidimetrischen oder Partikelzählverfahren, in Gegenwart eines Antiserums, das keine Antikörper enthält, die für einen der immunologischen Reaktionspartner spezifisch sind, dadurch gekennzeichnet, daß der Nachweis oder die Bestimmung in Gegenwart von 0.05 bis 2 ml/100 ml ®Tween 20 durchgeführt wird.

Als Antiserum eignen sich tierische Gammaglobuline oder hitzeaggregierte humane Gammaglobuline. Ein solches tierisches Gammaglobulin ist beispielsweise ein Anti-Schaf-Erythrozyten-Serum von einem Säugetier und vorzugsweise ein Anti-Schaf-Erythrozyten-Serum von Kaninchen. Solche Gammaglobuline sind auch Gammaglobulinfraktionen, die mittels bekannter Verfahren, beispielsweise Ammonsulfat Fällung oder Ionenaustauscher-Chromatographie gewonnen werden können. Als Partikel, die mit einem der Partner einer immunologischen Reaktion zur Verwendung in dem erfindungsgemäßen Verfahren beladen werden können, um sogenannte "Reagenzien" für diesen Zweck zu erhalten, sind beispielsweise Partikel von Latex-Dispersionen geeignet.

Solche Latex-Partikel sollten aus "nicht-filmbildenden" Polymerisaten bestehen. Unter nicht-filmbildend werden Polymer-Latexteilchen verstanden, die keinen Film unter den hier in Frage kommenden Anwendungsbedingungen bilden und nicht zusammenfließen. Polymerisate aus carbocyclischen aromatischen Monovinylidenmonomeren, wie Styrol, Vinyltoluol oder Vinylnaphthalin sowie Mischungen dieser Monomeren untereinander und/oder mit Methylmethacrylat und Acrylnitril sind bevorzugt. Besonders bevorzugte Saatdispersionen sind Polystyrol-Latices.

Das erfindungsgemäße Verfahren wird in Anwesenheit von 0.05 bis 2 g/100 ml ᴿTween 20 und/oder 0.5 bis 3 g/100 ml eines Neutralsalzes, vorzugswseise Kochsalz ausgeführt.

Ein Partner einer immunologischen Reaktion kann adsorptiv oder durch kovalente Bindung auf die Partikel aufgebracht und auf eine solche Weise "beladen" werden. Die Kuppelung der Partikel mit einem Antigen oder Antikörper kann nach einer bekannten Methode durchgeführt werden.

Bevorzugt werden die Partikel mit Antikörpern gegen Serumproteine wie Alpha-Fetoprotein (AFP), Myoglobin, Beta-2-Mikroglobulin oder Immunglobulin E, humane Hormone wie Human-Choriogonadotropin, Enzyme wie Pankreas-Lipase oder tierische Hormone wie Pregnant mare serum gonadotropin beladen.

Besonders bevorzugt ist ein erfindungsgemäßes Verfahren, worin Antikörper gegen Alpha-Fetoprotein (AFP) oder Immunglobulin E (IgE) kovalent an Latex-Partikel gebunden sind.

Wird AFP nach dem Verfahren des Standes der Technik wie zum Beispiel in der EP-A-0 080 614 beschrieben, also ohne Zusatz eines Antiserums, gemessen, so erhält man für Seren, die Rheumafaktoren enthalten, deutlich meßbare scheinbare Werte von AFP (Tab. 1), während bei erfindungsgemäßer Testdurchführung, besonders bei Verwendung von Anti-Schaf-Erythrozyten-Serum vom Kaninchen Ergebnisse mit völliger Übereinstimmung mit dem Enzymimmunoassay (EIA) erhalten werden.

Der Zusatz von Anti-Schaf-Erythrozyten-Serum vom Kaninchen stört nicht die Messung von tatsächlich vorhandenem AFP in entsprechenden Serumproben, wie Tabelle 2 zeigt.

Tabelle 1: Nephelometrische Bestimmung von AFP in 15
Seren, die Rheumafaktoren enthalten.

| RF-Gehalt (IU/ml) | Alpha-Fetoprotein-Gehalt (IU/ml) nephelometrisch | | |
|---|---|---|---|
| | im EIA | scheinbar nach St.d.T. | erfindungsgemäß Messung |
| 711 | * 3 a) | 19 | * 7 b) |
| 2768 | * 3 | 96 | * 7 |
| 474 | * 3 | 16 | * 7 |
| 578 | * 3 | 19 | * 7 |
| 251 | * 3 | 7 | * 7 |
| 543 | * 3 | * 7 b) | * 7 |
| 2760 | * 3 | 13 | * 7 |
| 446 | * 3 | 23 | * 7 |
| 178 | * 3 | 20 | * 7 |
| 2078 | * 3 | 46 | * 7 |
| 191 | * 3 | 18 | * 7 |
| 1626 | * 3 | 50 | * 7 |
| 158 | * 3 | 31 | * 7 |
| 248 | * 3 | 10 | * 7 |
| 513 | * 3 | 57 | * 7 |

*   bedeutet "kleiner als"

a) AFP-Gehalt ist sehr gering und daher nicht meßbar im Enzymimmunoassay.

b) Nicht auswertbar, aber Wert unterhalb des Meßbereiches korrekt wiedergefunden.

Alle Seren wurden auch in einem Enzym-Immuno-Assay (EIA) und in dem erfindungsgemäßen Verfahren geprüft. In den geprüften Seren ist die Konzentration an AFP gering und im EIA sogar nicht meßbar. Wie die Tabelle 1 zeigt, ergibt eine nephelometrische Bestimmung nach dem Stand der Technik scheinbare Konzentrationen an AFP (falsch positive Werte). Mit dem erfindungsgemäßen Verfahren geprüft, ergaben alle Seren Werte unterhalb des Meßbereiches, also in Übereinstimmung mit dem EIA keine falsch positiven Werte.

## Tabelle 2: Nephelometrische Bestimmung von AFP in 15 Seren, die AFP enthalten.

| AFP-Gehalt im EIA (IU/ml) | AFP-Gehalt erfindungsgemäß bestimmt (IU/ml) |
|---|---|
| 50,9 | 53,0 |
| 168,2 | 173,0 |
| 56,4 | 58,1 |
| 68,8 | 67,9 |
| 44,6 | 42,0 |
| 169,0 | 149,0 |
| 46,3 | 41,5 |
| 27,0 | 25,4 |
| 19,8 | 14,4 |
| 11,3 | 11,2 |
| 29,4 | 26,5 |
| 14,6 | 10,0 |
| 33,2 | 31,4 |
| 89,4 | 103,0 |
| 127,0 | 154,0 |

Alle Seren wurden auch in einem Enzym-Immuno-Assay (EIA) und in dem erfindungsgemäßen Verfahren geprüft. In den geprüften Seren ist die Konzentration an AFP in beiden Verfahren im Rahmen der dem Fachmann geläufigen leichten Abweichungen unterschiedlicher Testverfahren weitgehend übereinstimmend.

Gleichermaßen findet man für den IgE-Test, durchgeführt nach dem Stand der Technik falsch hohe IgE-Werte für Seren von Rheumapatienten (Tabelle 3) . In dieser Tabelle sind auch die Ergebnisse für die erfindungsgemäße Testdurchführung dargestellt. Hier zeigt sich ebenfalls die vorteilhafte Wirkung eines Zusatzes von Anti-Schaf-Erythrozyten-Serum vom Kaninchen. Auf diese Weise erhält man Ergebnisse, die eine gute Übereinstimmung mit denen des Enzymimmunoassays zeigen.

Tabelle 3: Nephelometrische Bestimmung von IgE in 15
Seren, die Rheumafaktoren enthalten.

| RF-Gehalt (IU/ml) | Immunglobulin E (IgE)-Gehalt (IU/ml) | | |
|---|---|---|---|
| | | nephelometrisch | |
| | im EIA | scheinbar nach St.d.T. | erfindungsgemäß |
| 474 | 44 | 102 | 47 |
| 2475 | 120 | 486 | 179 |
| 652 | 115 | 547 | 178 |
| 1501 | 342 | 515 | 293 |
| 2332 | 147 | 494 | 202 |
| 478 | 57 | 126 | 62 |
| 400 | 32 | 171 | 74 |
| 1940 | 124 | 483 | 176 |
| 1750 | 37 | 150 | 38 |
| 199 | 32 | 304 | 35 |
| 696 | 176 | 326 | 211 |
| 74 | 9 | 47 | *35 |
| 202 | 103 | 187 | 98 |
| 53 | 1 | *35 | *35 |
| 140 | 4 | 83 | *35 |

* bedeutet "kleiner als"

Ergebnis:

Nephelometrisch nach dem St.d.T. werden viel zu hohe Werte gefunden. Die mittlere Abweichung der Ergebnisse des nephelometrischen Testes von den Werten des Enzymimmunoassays beträgt etwa 370 %.

Nach dem erfindungsgemäßen Verfahren werden die Werte meist korrekt gefunden. Die mittlere Abweichung der Ergebnisse des erfindungsgemäßen nephelometrischen Testes von den Werten des Enzymimmunoassays beträgt in diesem Fall etwa 25 %.

Die Kupplung der genannten Reagenzienpartikel mit den Antigenen oder Antikörpern kann nach einer bekannten Methode durchgeführt werden.

Bevorzugt wird der Latex mit Antikörpern gegen Serumproteine wie Alpha-Fetoprotein, Myoglobin, Beta-2-Mikroglobulin oder Immunglobulin E, humane Hormone wie Human-Choriogonadotropin, Enzyme wie Pankreas-Lipase oder tierische Hormone wie Pregnant mare serum gonadotropin beladen.

Die verwendeten Antiseren werden durch Immunisieren von Tieren, besonders Kaninchen, Schafen und Ziegen mit einem Protein humanen oder tierischen Ursprungs, die das im Test zu bestimmende Protein nicht enthalten dürfen, hergestellt.

Beispiele sind: Anti-Human-IgG-Serum vom Kaninchen, Anti-Human-IgM-Serum vom Kaninchen, Anti-Schaf-Erythrozyten-Serum vom Kaninchen, Anti-Human-IgG-Serum vom Schaf, Anti-Kaninchen-Gammaglobulin-Serum vom Schaf. Besonders geeignet ist das Anti-Schaf-Erythrozyten-Serum vom Kaninchen. Die Immunisierung wurde nach bekannten Methoden durchgeführt. Immunisierungsdosis und -zeit ergeben sich aus der Inmunogenität und dem Molekulargewicht des Proteins.

Die verwendeten Antiköper-Lösungen enthalten keine Antikörper gegen das an die Latex-Partikel gebundene tierische Antiserum. Sie sind gewöhnlich von derselben Tierspezies.

Ein solches Antiserum oder eine solche Gammaglobulin-Lösung wird der Lösung, in der ein Antigen oder ein Antikörper bestimmt oder nachgewiesen werden soll, zugesetzt.

Ein solches Antiserum wird in einer Menge zugegeben, daß es in einer Konzentration zwischen 50 und 0,05 ml/100 ml in der Testmischung vorliegt. Vorteilhafter ist eine Konzentration zwischen 10 und 0,1 ml/100 ml im Testansatz. Besonders vorteilhaft ist eine Konzentration zwischen 2 und 0,2 ml/100 ml im Testansatz.

Zusätzlich ist eine Konzentration von 0,05 bis 2 ml/100 ml des nicht-ionischen Detergenzes Eikosaoxyethylen-sorbitanlaurylsäureester ($^R$Tween 20) in der Meßküvette.

Das erfindungsgemäße Verfahren ist zum Nachweis von allen immunologisch aktiven Substanzen, die im Blut (Serum, Plasma) von Säugetieren, insbesondere vom Menschen, enthalten sind, anwendbar. Beispiele solcher immunologisch aktiver Substanzen sind Serumproteine.

Für das erfindungsgemäße Verfahren wird ein Mittel in die Testküvette gegeben. Dieses Mittel enthält 90 bis 0,1 ml/100 ml eines Antiserums, das keine Antikörper enthält, die für einen der immunologischen Reaktionspartner spezifisch sind. Besonders vorteilhaft enthält das Mittel 20 bis 1 ml/100 ml des genannten Antiserums. Zusätzlich enthält das Mittel 50 bis 0,1 ml/100 ml $^R$Tween 20, vorteilhaft 20 bis 0,5 ml/100 ml $^R$Tween 20.

Beispiel 1

1. Herstellung von Saatpolymerisat für den Latex

In ein zylindrisches Glasgefäß, ausgestattet mit Gaseinlaß- und Gasauslaßrohr sowie einem Magnetrührstab wurden 310 ml stickstoffgesättigtes bidestilliertes Wasser gegeben. Man gab 500 mg Natriumstearat hinzu und löste dieses durch Rühren. Weiterhin wurden 1,5 ml Ammoniak (25 g/100 ml) hinzugegeben. Der pH wurde kontrolliert und betrug 11,09. Durch mehrfaches Evakuieren und Füllen mit Stickstoff wurde das Polymerisationsgefäß Sauerstoff-frei gemacht. Unter ständigem Rühren wurde die Detergenz-Lösung mit Hilfe eines Wasserbades auf +70°C erwärmt. Anschliessend gab man 90 ml frisch destilliertes Styrol unter Stickstoff mit Hilfe eines Tropftrichters mit Druckausgleich in das Polymerisationsgefäß. Bei +70°C wurde weitere 15 Minuten zur Emulgation des Styrols gerührt. Anschließend wurde die Temperatur auf +90°C angehoben und eine weitere Stunde gerührt. Danach wurden 67,5 mg Kaliumperoxydisulfat gelöst in 50 ml stickstoffgesättigtem destilliertem Wasser hinzugegeben. Man ließ 130 Minuten bei +90°C rühren. Das Polystyrol wurde durch einen Faltenfilter gegeben. Das filtrierte Polystyrol wurde gegen 10 Liter Ammoniumhydrogencarbonat-Lösung (0,01 g/100 ml NH$_4$HCO$_3$; 0,01 g/ 100 g NaN$_3$; mit 10,5 ml Ammoniak 25 g/100 ml in 10 l auf pH 10 eingestellt) 50 Stunden lang dialysiert. Man erhielt nach Dialyse 410 ml Polymerisat mit einem Trockengewicht von 17,9 g / 100 ml.

2. Polymerisation von 2-Hydroxypropylmethacrylat (HPM) auf Polystyrolkerne

Die Polymerisation erfolgte in einem Gefäß ähnlich wie im Beispiel 1 beschrieben. Man stellte eine Mischung von 22,4 ml Polystyrol-Latex mit einem Feststoffgehalt von 17,9 g/100 g und 56,7 ml destilliertem Wasser sowie 50 mg Natriumdodecylsulfat her. Diese wurde in das Polymerisationsgefäß gegeben und der Sauerstoff entfernt. Weiterhin wurden 1 ml einer Kaliumperoxidisulfatlösung (16 mg/ml in destilliertem Wasser) hinzugegeben und der Ansatz auf +70°C erhitzt.

Man stellte eine Mischung aus 0,4 ml Styrol, 0,4 ml Methacrylamidoacetaldehyd-di-n-pentylacetal, 0,025 ml Methacrylsäure und 0,2 ml 2-Hydroxypropyl-methacrylat (HPM) her. Die Monomerenmischung wurde zur kräftig gerührten Polystyrol-Latex-Suspension bei +70°C während 60 Minuten langsam hinzugetropft. Anschließend wurde weitere 4 Stunden bei gleicher Temperatur weitergerührt.

Nach Abkühlung auf Raumtemperatur und Filtration durch ein Faltenfilter erhielt man 73 ml des Polymerisats. Man dialysierte anschließend etwa 20 Stunden gegen NaHCO$_3$-Puffer (0,25 g/l, pH 8-8,2). Man erhielt 87 ml einer Latex-Dispersion mit einem Feststoffgehalt von 5,1 g/ 100 g.

3. Bindung an AFP-Antikörpern an ein Polymerisat

An ein Polymerisat unter Verwendung von 2-Hydroxypropyl-methacrylat hergestellt nach 2. wurden AFP-Antikörper gebunden, wie unten angegeben. Das jeweils eingesetzte Polymerisat wurde mit destilliertem Wasser auf einen Feststoffgehalt von 4 g/ 100 g verdünnt. Ein Antiserum, gewonnen durch Immunisierung von Kaninchen mit gereinigtem AFP, wurde nach bekannten Verfahren affinitätschromatographisch gereinigt. Es wurde anschließend konzentriert bis ein Proteingehalt von 10 mg/ml erreicht war.

3,4 ml des o.g. Polymerisates wurden mit 0,34 ml der AFP-Antikörper-Lösung gemischt. Dann wurden 0,17 ml einer 20 ml/100 ml wäßrigen Lösung von Eikosaoxyethylen-sorbitanlaurinsäureester ($^R$Tween 20) hinzugegeben und das ganze nochmals gemischt. Hierzu gab man 0,05 ml 1 N HCl, so daß ein pH-Wert von etwa 2 erreicht wurde. Nach einer Inkubationszeit von 30 Minuten bei Raumtemperatur setzte man 0,85 ml gesättigte wäßrige Dinatriumhydrogenphosphat-Lösung (25 mg/ml) hinzu und mischte gut durch. Anschließend erfolgte eine Inkubation von einer Stunde bei Raumtemperatur.

Dieser Beladungsansatz wurde sodann 30 Minuten mit etwa 50.000 g zentrifugiert. Der Überstand wurde verworfen. Der Rückstand wurde in 5 ml eines Glycin-NaCl-Puffers (0,1 mol/l Glycin, 0,17 mol/l NaCl, 0,5 ml/100 ml Eikosaoxyethylen-sorbitan-laurinsäureester (RTween 20), pH 8,2) resuspendiert. Anschließend erfolgte eine Ultraschall-Behandlung für 2 Sekunden. Das so redispergierte Reagenz wurde im Volumenverhältnis 1:60 mit dem zuvor genannten Glycin-NaCl-Puffer verdünnt.

Beispiel 2

Bindung von Anti-IgE Antikörpern an ein Polymerisat

An ein Polymerisat, unter Verwendung von 2-Hydroxypropylmethacrylat, hergestellt nach Beispiel 1, wurden Anti-IgE- Antikörper gebunden. Das jeweils eingesetzte Polymerisat wurde mit destilliertem Wasser auf einen Feststoffgehalt von 4 g/100 g verdünnt. Ein Antiserum, gewonnen durch Immunisierung von Kaninchen mit aufgereinigtem IgE, wurde nach bekannten Verfahren affinitätschromatographisch aufgereinigt. Es wurde anschließend aufkonzentriert, bis ein Proteingehalt von 10 mg/ml erreicht war.

3,4 ml des obengenannten Polymerisates wurden mit 0,34 ml der Anti-IgE-Antikörper-Lösung gemischt. Dann wurden 0,17 ml einer 20 ml/100 ml wäßrigen Lösung von Eikosa-oxyethylen-sorbitanlaurylsäureester (RTween 20) hinzugegeben und das Ganze nochmals gemischt. Hierzu gaben wir 0,05 ml 1N HCl, so daß ein pH-Wert von etwa 2 erreicht wurde. Nach einer Inkubationszeit von 30 Minuten bei Raumtemperatur setzte man 0,85 ml gesättigte wässrige Dinatriumhydrogenphosphat-Lösung (pH 6,5) und 0,85 ml wässrige Natriumcyanborhydrid-Lösung (25 mg/ml) hinzu und mischte gut durch. Anschließend erfolgte eine Inkubation von einer Stunde bei Raumtemperatur.

Dieser Beladungsansatz wurde sodann 30 Minuten mit etwa 50.000 g zentrifugiert. Der Überstand wurde verworfen. Der Rückstand wurde in 5 ml eines Glycin-NaCl-Puffers (0,1 mol/l Glycin, 0,17 mol/l NaCl, 0,5 ml/100 ml Eikosaoxyethylen-sorbitan-laurinsäureester (RTween 20), pH 8,2) resuspendiert. Anschließend erfolgte eine Ultraschall-Behandlung für 2 Sekunden. Das so redispergierte Reagenz wurde im Volumenverhältnis 1:80 mit dem zuvor genannten Glycin-NaCl-Puffer verdünnt.

Beispiel 3

Messung von AFP-Konzentrationen in Serumproben

Das nach Beispiel 1 durch Bindung von Anti-AFP-Antikörpern an Latexpräparationen hergestellte Reagenz zur Bestimmung von AFP wurde zur Messung von AFP in Seren eingesetzt. Als Standard wurde das Alpha-Fetoprotein Standard-Serum (human) für die Immunpräzipitation mit einer AFP-Konzentration von 322000 ng/ml (Fa. Behringwerke AG, Marburg, FRG) verwendet. Der Standard wurde in einem AFP-freien Serumpool auf 1000 ng/ml verdünnt. Diese Verdünnung wurde in dem AFP-freien Serumpool stufenweise auf jeweils das doppelte Volumen weiter verdünnt. Man erhielt so eine Standardreihe mit abnehmenden AFP-Konzentrationen. Die zu bestimmenden Patientensera wurden 1:5 in einem Phosphat-Kochsalz-Puffer (1,2 g/100 ml NaCl,1,3 g/100 ml $Na_2HPO_4$, 0,2 g/100 ml $NaH_2PO_4$) verdünnt. Zur Messung wurden 80 µl Patientenserum-Verdünnung, beziehungsweise Standardserum-Verdünnung, mit 160 µl eines Reaktionspuffers (1,2 g/100 ml NaCl, 1,3 g/ 100 ml $Na_2HPO_4$, 0,2 g/100 ml $NaH_2PO_4$, 5,6 g/100 ml Polyethylenglykol 6000) sowie 30 µl des Antiserums gegen Schaf-Erythrozyten vom Kaninchen 1:8 verdünnt in Phosphat-Kochsalz-Puffer (1,2 g/100 ml NaCl, 1,3 g/100 ml $Na_2HPO_4$, 0,2 g/100 ml $NaH_2PO_4$) mit 5 ml/100 ml RTween 20 sowie 60 µl AFP-Reagenz (Beispiel 1.3) 12 Minuten bei Raumtemperatur inkubiert. Die Ergebnisse wurden dann in einem Nephelometer (beispielsweise dem der Behringwerke AG) gemessen. Die Referenzkurve für die Messung des Standardserums wurde aufgezeichnet und daran wurden die Meßwerte für die Patientenseren ausgewertet.

Beispiel 4

Messung von IgE-Konzentrationen in Serumproben

Das nach Beispiel 2 hergestellte Reagenz wurde zur Messung von IgE in Patientenseren eingesetzt. Dieser IgE-Standard enthielt 1000 IU/ml. Der Standard wurde in einem IgE-freien Serumpool stufenweise auf jeweils das doppelte Volumen verdünnt. Man erhielt so eine Standardreihe mit abnehmenden IgE-Konzentrationen.

Die zu bestimmenden Patientensera wurden in einem Phosphat-Kochsalz-Puffer (1,2 g/100 ml NaCl, 1,3 g/100 ml $Na_2HPO_4$, 0,2 g/100 ml $NaH_2PO_4$) verdünnt. Zur Messung wurden 80 µl Patientenserum-Verdünnung, beziehungsweise Standardserum-Verdünnung mit 150 µl eines Reaktionspuffers (1,2 g/100 ml NaCl, 1,3 g/100 ml $Na_2HPO_4$, 0,2 g/100 ml $NaH_2PO_4$, 5,6 g/100 ml Polyethylenglykol 6000) sowie 20 µl des Antiserums gegen Schaf-Erythrozyten vom Kaninchen 1:30, verdünnt in Phosphat-Kochsalz-Puffer (1,2 g/100 ml NaCl, 1,3 g/100 ml $Na_2HPO_4$, 0,2 g/100 ml $NaH_2PO_4$) mit 4 ml/100 ml [R]Tween 20 sowie 75 µl IgE-Reagenz (Beispiel 3) 12 Minuten bei Raumtemperatur inku-biert. Die Ergebnisse wurden dann in einem Nephelometer (beispielsweise dem der Behringwerke AG) gemessen. Die Referenzkurve für die Messung des Standardserums wurde aufgezeichnet und daran wurden die Meßwerte für die Patientenseren ausgewertet.

## Patentansprüche

1. Verfahren zum Nachweis oder zur Bestimmung eines Partners einer partikelverstärkten immunologischen Reaktion nach einem nephelometrischen, turbidimetrischen oder Partikelzählverfahren, in Gegenwart eines Antiserums, das keine Antikörper enthält, die für einen der immunologischen Reaktionspartner spezifisch sind, dadurch gekennzeichnet, daß der Nachweis oder die Bestimmung in Gegenwart von 0,05 bis 2 ml/100 ml [R]Tween 20 (Polyolyoxyethylen(20)sorbitanmonolaurat) durchgeführt wird.

2. Verfahren nach Anspruch 1, dadurch gekennzeichnet, daß das Antiserum eine wäßrige Lösung eines Gammaglobulins ist.

3. Verfahren nach Anspruch 1, dadurch gekennzeichnet, daß das Antiserum ein Anti-Schaf-Erythrozyten-Serum von einem Säugetier ist.

4. Verfahren nach Anspruch 1, dadurch gekennzeichnet, daß das Antiserum ein Anti-Schaf-Erythrozyten-Serum von einem Kaninchen ist.

5. Verfahren nach Anspruch 1, dadurch gekennzeichnet, daß die Reaktion unter Verwendung eines Latexreagenzes mit kovalent gebundenem Antigen, Antikörper oder Hapten durchgeführt wird.

6. Mittel zur Durchführung des Verfahrens nach Anspruch 1 enthaltend 0.1 bis 90 ml/100 ml eines Antiserums, das keine Antikörper enthält, die für einen der immunologischen Reaktionspartner spezifisch sind und 0.1 bis 50 ml/100 ml [R]Tween 20.

7. Mittel nach Anspruch 6 enthaltend 1 - 20 ml/100 ml eines Antiserums, das keine Antikörper enthält, die für einen der immunologischen Reaktionspartner spezifisch sind.

8. Mittel nach Anspruch 6 oder 7 enthaltend 0.5 - 3 g/100ml eines Neutralsalzes.

9. Mittel nach Anspruch 8 enthaltend NaCl als Neutralsalz.

## Claims

1. A method for the detection or determination of a participant in a particle-amplified immunological reaction by a nephelometric, turbidimetric or particle-counting method, in the presence of an antiserum which contains no antibodies specific for one of the immunological reaction participants, which comprises carrying out the detection or determination in the presence of 0.05 to 2 ml/100 ml of [R]Tween 20 (polyoxyethylene (20) sorbitol mono-laurate).

2. The method as claimed in claim 1, wherein the antiserum is an aqueous solution of a gamma-globulin.

3. The method as claimed in claim 1, wherein the antiserum is an anti-sheep erythrocyte serum from a mammal.

4. The method as claimed in claim 1, wherein the antiserum is an anti-sheep erythrocyte serum from a rabbit.

5. The method as claimed in claim 1, wherein the reaction is carried out using a latex reagent with a covalently bound antigen, antibody or hapten.

6. An agent for carrying out the method as claimed in claim 1, containing 0.1 to 90 ml/100 ml of an antiserum which contains no antibodies specific for one of the immunological reaction participants, and 0.1 to 50 ml/100 ml of ®Tween 20.

7. The agent as claimed in claim 6, containing 1-20 ml/100 ml of an antiserum which contains no antibodies specific for one of the immunological reaction participants.

8. The agent as claimed in claim 6 or 7, containing 0.5-3 g/100 ml of a neutral salt.

9. The agent as claimed in claim 8, containing NaCl as neutral salt.


## Revendications

1. Procédé de détection ou de dosage d'un partenaire d'une réaction immunologique renforcée par des particules, procédé néphélémétrique, turbidimétrique ou de comptage de particules, en présence d'un antisérum ne contenant pas d'anticorps spécifiques pour l'un des parternaires de la réaction immunologique, caractérisé en ce que la détection ou le dosage est effectué en présence de 0,05 à 2 ml/100 ml de Tween(R) 20 (sorbitanemonolaurate d'eicosa-oxyéthylène).

2. Procédé selon la revendication 1, caractérisé en ce que l'antisérum est une solution aqueuse d'une gammaglobuline.

3. Procédé selon la revendication 1, caractérisé en ce que l'antisérum est un sérum de mammifère anti-érythrocytes de mouton.

4. Procédé selon la revendication 1, caractérisé en ce que l'antisérum est un sérum de lapin anti-érythrocytes de mouton.

5. Procédé selon la revendication 1, caractérisé en ce qu'on effectue la réaction en utilisant un réactif latex à antigène, anticorps ou haptène fixé par covalence.

6. Agent pour réaliser le procédé selon la revendication 1, qui renferme 0,1 à 90 ml/100 ml d'un antisérum ne contenant pas d'anticorps spécifiques pour l'un des partenaires de la réaction immunologique, et 0,1 à 50 ml/100 ml de Tween(R) 20.

7. Agent selon la revendication 6, qui renferme 1 à 20 ml/100 ml d'un antisérum ne contenant pas d'anticorps spécifiques pour l'un des partenaires de la réaction immunologique.

8. Agent selon la revendication 6 ou 7, qui renferme 0,5 à 3 g/100 ml d'un sel neutre.

9. Agent selon la revendication 8, qui renferme NaCl comme sel neutre.